# EUROPEAN PATENT APPLICATION

(11) **EP 4 851 144 A1**
(43) Date of publication of application: **09.12.2026**
(21) Application number: 25749044.1
(22) Date of filing: 31.01.2025
(51) Int. Cl.: C07D 333/36, A61K 31/381, A61P 11/00, C07C 323/60

(54) **USE OF L-ERDOSTEINE COMPOUND**

(30) Priority: 01.02.2024 KR 20240015610
(71) Applicant: Daebong LS Co., Ltd., Namdong-gu Incheon 21697 (KR)
(72) Inventor: PARK, Jin Oh, Seoul 06276 (KR); PARK, Eun Ju, Hwaseong-si Gyeonggi-do 18425 (KR); JI, Hyun, Siheung-si Gyeonggi-do 14923 (KR); LEE, Chan Hyung, Chungju-si Chungcheongbuk-do 27369 (KR)
(74) Representative: Ter Meer und Partner mbB
(86) International application number: PCT/KR2025/001563
(87) International publication number: WO 2025/165141

(57) **Abstract**

The present invention relates to a use of L-erdosteine and, more specifically, to a use of L-erdosteine in the production of a pharmaceutical agent for at least one selected from among: mucolysis and expectoration in acute and chronic respiratory diseases; treating or ameliorating upper respiratory tract inflammation; treating or preventing acute and chronic bronchitis; treating or preventing cough; treating or preventing asthma; treating or preventing pneumonia; preventing pulmonary fibrosis; preventing bronchial and alveolar destruction; treating or preventing chronic obstructive pulmonary disease; enhancing resistance to bacterial infections of the upper respiratory tract; and ameliorating side effects caused by smoking. The present invention rapidly increases the blood concentration of Met-I, which is a metabolically active form of erdosteine, thereby exhibiting a rapid and excellent effect without toxicity, and thus has the effect of achieving the same effect at a lower dose than existing racemic compounds. In addition, since a lower dose can be used as compared to existing racemic compounds, the present invention also has the effect of reducing expected potential side effects.

## Description

### Technical Field

The present invention relates to a use of L-erdosteine.

### Background Art

Erdosteine is a compound having the IUPAC name of 2-((2-oxo-2-((2-oxotetrahydrothiophen-3-yl)amino)ethyl)thio)acetic acid, and a representative commercial product thereof is Erdos^{™}. Erdosteine is an ingredient having mucolytic and expectorant effects, and is widely used as a therapeutic agent for acute and chronic respiratory diseases, including bronchitis. In particular, erdosteine is known to have actions that other expectorants do not have, such as a ciliary motility-promoting action, an antibacterial action, an anti-inflammatory action, an antioxidant action, and a synergistic action when used in combination with antibiotics. More specifically, erdosteine inhibits the oxidation of α1-antitrypsin induced by smoking to prevent bronchial and alveolar destruction, increases physiological antibacterial substances in the body such as immunoglobulin A and lysozyme, which are respiratory immune substances, thereby enhancing the resistance against bacteria, and is also known to be effective even for chronic obstructive pulmonary disease (COPD). Such erdosteine, when administered orally, is absorbed in the small intestine, metabolized into metabolites in the liver, and excreted through the kidneys (absorption is not affected by food; the maximum blood concentration is reached in about 1.4 hours, and the maximum blood concentration is about 2.5 µg/ml). In other words, erdosteine is a kind of prodrug that is activated by metabolism by hepatic enzymes after absorption. Three types of erdosteine metabolites are known, namely Met-I, Met-II, and Met-III, among which the pharmacologically important active metabolite is Met-I (also referred to as Metabolite-1 or Metabolite-I) compound represented by Chemical Formula I below (including the L-form, the D-form, and the racemate thereof with reference to the asymmetric carbon).

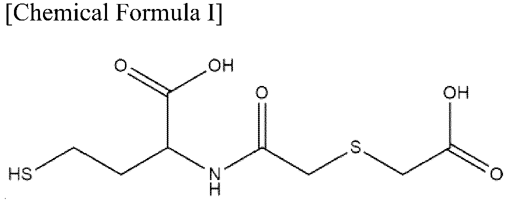

The Met-I compound has two blocked sulfhydryl groups that are released after first-pass metabolism, thereby regulating mucus production and viscosity and increasing mucus excretion, and in particular, a free thiol group thereof can exhibit an inhibitory action against the action of free radicals generated by cigarette smoke and the like, so that the Met-I compound is recognized as a substantial active form of erdosteine. Therefore, a substance capable of rapidly increasing the blood concentration of Met-I, the active metabolite of erdosteine, to a high level would serve as a highly desirable prodrug. However, the erdosteine that is commonly used at present is a racemic compound in which the D-form and the L-form are mixed, and the racemic compound is used as a pharmaceutical agent without separation of the enantiomers.

### [Prior Art Document]

(Non-patent document 1) Mario Cazzola et al., "Multifaceted Beneficial Effects of Erdosteine: More than a Mucolytic Agent," Drugs (2020) 80:1799-.1809, https://doi.org/10.1007/s40265-020-01412-x

### Disclosure

### Technical Problem

An object of the present invention is to provide an L-erdosteine compound, which can rapidly raise the blood concentration of Met-I, the active metabolite, to a high level so as to exhibit excellent effects, while being non-toxic and being capable of producing the same effect even at a lower dose than that of a conventional racemic compound, and a use thereof.

### Technical Solution

The present invention has been devised to solve the problems of the prior art described above.

The present invention provides a use of an L-erdosteine compound represented by Chemical Formula 1 below in the manufacture of a medicament for one or more selected from mucolysis and expectoration in acute and chronic respiratory diseases, treatment or amelioration of upper respiratory tract inflammation, treatment or prevention of acute and chronic bronchitis, treatment or prevention of cough, treatment or prevention of asthma, treatment or prevention of pneumonia, prevention of pulmonary fibrosis, prevention of bronchial and alveolar destruction, treatment or prevention of chronic obstructive pulmonary disease (COPD), increase of resistance to bacterial infections of upper respiratory tract, and amelioration of side effects caused by smoking.

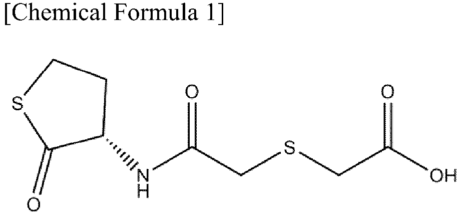

In addition, the present invention provides a use of an L-erdosteine compound as a prodrug of the compound represented by Chemical Formula 2 below.

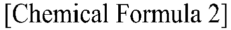

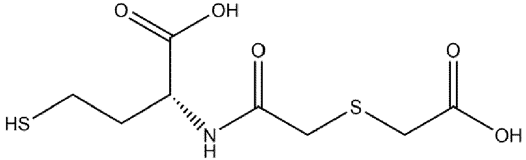

The present invention also provides a pharmaceutical composition including an L-erdosteine compound as an active ingredient, for one or more uses selected from mucolysis and expectoration in acute and chronic respiratory diseases, treatment or amelioration of upper respiratory tract inflammation, treatment or prevention of acute and chronic bronchitis, treatment or prevention of cough, treatment or prevention of asthma, treatment or prevention of pneumonia, prevention of pulmonary fibrosis, prevention of bronchial and alveolar destruction, treatment or prevention of COPD, increase of resistance to bacterial infections of upper respiratory tract, and amelioration of side effects caused by smoking.

In addition, the present invention provides a pharmaceutical composition including the L-erdosteine compound at a daily dose of 10 to 200 mg/kg.

In addition, the present invention provides a pharmaceutical composition including 50 to 200 mg of the L-erdosteine compound and a pharmaceutically acceptable excipient.

### Advantageous Effects

The present invention has the effect of rapidly raising the blood concentration of Met-I, the metabolic active form of erdosteine, so as to rapidly exhibit excellent effects, while being non-toxic and being capable of producing the same effect even at a lower dose than that of a conventional racemic compound. In addition, since the same effect can be obtained even when using a smaller dose than the conventional racemic compound, there is an effect of reducing the anticipated potential side effects.

### Brief Description of Drawings

FIG. 1 is a photograph showing the results of a solubility test of the L-form, the D-form, and the racemate of erdosteine.
FIG. 2 is a graph showing the results of the microsomal and S9 stability assay tests.
FIGS. 3 to 5 show the results of animal tests on the pharmacokinetics after administration of L-erdosteine and racemic erdosteine.
FIGS. 6 to 9 show the results of evaluating the bronchial relaxation effect when racemic erdosteine and L-erdosteine were applied to bronchi excised from guinea pigs.
FIGS. 10 and 11 show the results of evaluating cytotoxicity and the mRNA expression level of MUC5AC in the expectorant effect test of L-erdosteine and racemic erdosteine, respectively.
FIGS. 12 and 13 show, respectively, the sections from the histopathological examination and the analysis results thereof in the pulmonary inflammation-inhibitory effect test of L-erdosteine and racemic erdosteine.
FIG. 14 shows the ROMO1 analysis results in the respiratory inflammation-alleviating effect test of L-erdosteine and racemic erdosteine.

### Best Mode for Carrying Out the Invention

Hereinafter, the present invention will be described in detail.

In the present invention, the compound includes not only the compound itself but also a pharmaceutically acceptable salt thereof, a pharmaceutically acceptable hydrate or solvate thereof, and a pharmaceutically acceptable hydrate or solvate of a pharmaceutically acceptable salt thereof.

One aspect of the present invention relates to
a use of an L-erdosteine compound.

The L-erdosteine compound is a compound represented by Chemical Formula 1 below, and has never been used for the prevention or treatment of disease.

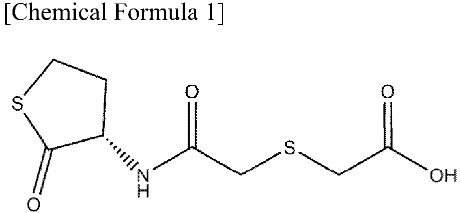

In other words, conventionally, only D,L-erdosteine (racemic erdosteine) has been used for the prevention or treatment of disease, but the L-erdosteine compound alone has never been used. Accordingly, the present invention provides a use of the L-erdosteine compound for the prevention or treatment of disease.

The use for the prevention or treatment of disease may be exemplified by one or more selected from mucolysis and expectoration in acute and chronic respiratory diseases, treatment or amelioration of upper respiratory tract inflammation, treatment or prevention of acute and chronic bronchitis, treatment or prevention of cough, treatment or prevention of asthma, treatment or prevention of pneumonia, prevention of pulmonary fibrosis, prevention of bronchial and alveolar destruction, treatment or prevention of chronic obstructive pulmonary disease (COPD), increase of resistance to bacterial infections of upper respiratory tract, and amelioration of side effects caused by smoking.

In addition, L-erdosteine may be a kind of prodrug for an active metabolite that is activated by metabolism by hepatic enzymes after absorption. L-erdosteine may be a prodrug of the Met-I compound represented by Chemical Formula 2 below, which has two blocked sulfhydryl groups that are released after first-pass hepatic metabolism, thereby regulating mucus production and viscosity and increasing mucus excretion, and in particular, a free thiol group thereof can exhibit an inhibitory action against the action of free radicals generated by cigarette smoke and the like, and therefore, Met-I is recognized as a substantial active form of erdosteine.

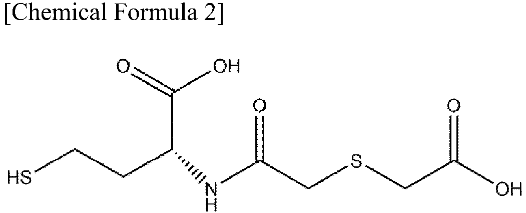

Another aspect of the present invention relates to
a pharmaceutical composition including an L-erdosteine compound as an active ingredient,
for one or more uses selected from mucolysis and expectoration in acute and chronic respiratory diseases, treatment or amelioration of upper respiratory tract inflammation, treatment or prevention of acute and chronic bronchitis, treatment or prevention of cough, treatment or prevention of asthma, treatment or prevention of pneumonia, prevention of pulmonary fibrosis, prevention of bronchial and alveolar destruction, treatment or prevention of COPD, increase of resistance to bacterial infections of upper respiratory tract, and amelioration of side effects caused by smoking

The pharmaceutical composition may include the L-erdosteine compound at a daily dose of 10 to 200 mg/kg, preferably 15 to 150 mg/kg, more preferably 20 to 120 mg/kg, and still more preferably 25 to 100 mg/kg.

The pharmaceutical composition may include 50 to 200 mg of the L-erdosteine compound and a pharmaceutically acceptable excipient. The single dose of the conventional erdosteine compound was about 300 mg, but the dose of the compound of the present invention may be slightly reduced.

Furthermore, as will be understood through the examples and experimental examples described later, the L-erdosteine compound is converted into the metabolic active compound rapidly and at a high concentration compared to the conventionally known D,L-erdosteine compound (racemic erdosteine).

The L-erdosteine compound of the present invention may be administered by the oral, parenteral, and topical routes as described below.

### Oral administration (P.O.)

The compound of the present invention may be administered orally, that is, into the oral cavity, and the oral cavity is a concept that includes swallowing. By oral administration, the compound of the present invention may enter the gastrointestinal tract, or may be absorbed directly into the bloodstream from the mouth, such as by buccal or sublingual administration. Compositions suitable for oral administration may be in a solid, liquid, gel, or powder form, and may have dosage forms such as tablets, lozenges, capsules, granules, and powders. Compositions for oral administration may optionally be enteric-coated, and through enteric coating, may exhibit delayed or sustained release. In other words, the composition for oral administration according to the present invention may be a dosage form having an immediate or modified release pattern. Liquid dosage forms may include solutions, syrups, and suspensions, and such liquid compositions may be contained within soft or hard capsules. Such dosage forms may include a pharmaceutically acceptable carrier, for example, water, ethanol, polyethylene glycol, cellulose, or an oil. The dosage form may also include one or more emulsifiers and/or suspending agents. In a tablet dosage form, the drug, which is the active ingredient, may be present in an amount of about 0.05% to about 95% by weight based on the total weight of the tablet, and more generally about 2% to about 50% by weight of the formulation. In addition, the tablet may contain a disintegrant in an amount of about 0.5% to about 35% by weight, and more generally about 2% to about 25% by weight of the formulation. Examples of the disintegrant include lactose, starch, sodium starch glycolate, crospovidone, croscarmellose sodium, maltodextrin, or mixtures thereof, but are not limited thereto. Suitable lubricants included for manufacturing the tablet may be present in an amount of about 0.1% to about 5% by weight, and talc, silicon dioxide, stearic acid, calcium, zinc or magnesium stearate, sodium stearyl fumarate, and the like may be used as the lubricant, but the present invention is not limited to these kinds of additives. As a binder for manufacturing the tablet, gelatin, polyethylene glycol, sugar, gum, starch, polyvinylpyrrolidone, hydroxypropyl cellulose, hydroxypropyl methylcellulose, and the like may be used, and as a suitable diluent for manufacturing the tablet, mannitol, xylitol, lactose, dextrose, sucrose, sorbitol, starch, microcrystalline cellulose, and the like may be used, but the present invention is not limited to these kinds of additives. A solubilizer that may optionally be included in the tablet may be used in an amount of about 0.1% to about 3% by weight based on the total weight of the tablet, and for example, polysorbate, sodium lauryl sulfate, sodium dodecyl sulfate, propylene carbonate, diethylene glycol monoethyl ether, dimethyl isosorbide, polyoxyethylene-glycolated natural or hydrogenated castor oil, HCO^{TM} (Nikkol), oleyl esters, Gelucire^{TM}, caprylic/capric mono/diglyceride, sorbitan fatty acid esters, Solutol HS^{TM}, and the like may be used in the pharmaceutical composition according to the present invention, but the present invention is not limited to these specific kinds of solubilizers.

### Parenteral administration

The compound of the present invention may be administered directly into the bloodstream, muscle, or internal organs. Suitable methods for parenteral administration include intravenous, intramuscular, subcutaneous, intraarterial, intraperitoneal, intrathecal, and intracranial injection. Suitable devices for parenteral administration include injectors (including needle and needleless syringes) and infusion methods. Compositions for parenteral administration may be dosage forms having an immediate or modified release pattern, and the modified release pattern may be a delayed or sustained release pattern. Most parenteral dosage forms are liquid compositions, and such liquid compositions are aqueous solutions containing the drug component, salts, buffers, isotonic agents, and the like according to the present invention. Parenteral dosage forms may also be prepared in a dried form (e.g., lyophilized) or as a sterile non-aqueous solution. These dosage forms may be used together with a suitable vehicle such as sterile water. Solubility-enhancing agents may also be used in the preparation of the parenteral solution.

### Topical administration

The compound of the present invention may be administered topically to the skin or transdermally. Dosage forms for such topical administration include lotions, solutions, creams, gels, hydrogels, ointments, foams, implants, patches, and the like. Pharmaceutically acceptable carriers for topical dosage forms may include water, alcohol, mineral oil, glycerin, polyethylene glycol, and the like. Topical administration may also be performed by electroporation, iontophoresis, phonophoresis, and the like. Compositions for topical administration may be dosage forms having an immediate or modified release pattern, and the modified release pattern may be a delayed or sustained release pattern.

Hereinafter, the present invention will be described in more detail with reference to examples and experimental examples. However, it is clearly noted that the following examples and experimental examples are only for the detailed description of the invention and are not intended to limit the scope of the claims thereby.

### Examples

### [Preparation of (R)-2-methoxy-N-((R)-2-oxotetrahydrothiophen-3-yl)-2-phenylacetamide]

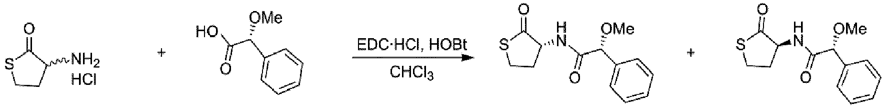

36 g of D,L-homocysteine thiolactone hydrochloride and 3.4 L of chloroform were introduced, and then the resulting mixture was cooled to 5 °C. 33.2 g of triethylamine was introduced into the reactor while maintaining 5 °C. 38 g of 1-hydroxybenzotriazole, 43.3 g of S-(+)-2-methoxyphenylacetic acid, and 54 g of 1-(3-dimethylaminopropyl)-3-ethylcarbodiimide hydrochloride were introduced into the reactor while maintaining 5 °C. While maintaining 0 to 5°C, the resulting mixture was stirred for 7 hours and 30 minutes, and then distilled water was introduced to terminate the reaction. The resulting mixture was washed with a 5% sodium bicarbonate solution, a 2 M hydrochloric acid solution, and a saturated sodium chloride solution, dried over magnesium sulfate, and concentrated under reduced pressure. The crude product was purified by column chromatography (silica, ethyl acetate/hexane).

### [Preparation of (R)-3-aminodihydrothiophen-2(3H)-one]

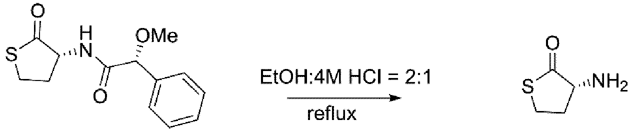

20 g of (*R*)-2-methoxy-*N*-((*R*)-2-oxotetrahydrothiophen-3-yl)-2-phenylacetamide and 1.2 L of a solution of ethanol:4 M HCl = 2:1 were introduced at room temperature. The temperature was raised, and the resulting mixture was refluxed and stirred for 25 hours, and then cooled to room temperature. Distilled water and toluene were added to remove impurities into the organic layer. The aqueous layer was concentrated under reduced pressure. The crude product was recrystallized using isopropyl alcohol to obtain 7.24 g (55.6%) of (R)-3-aminodihydrothiophen-2(3H)-one.

### [Preparation of L-erdosteine]

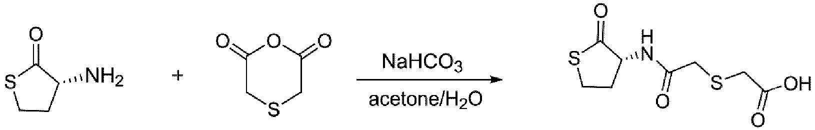

9 g of (*R*)-3-aminodihydrothiophen-2(3*H*)-one, 21.6 ml of distilled water, and 18 ml of acetone were introduced at room temperature.

After cooling to 0 to 10 °C, 4.92 g of sodium bicarbonate was introduced, and the resulting mixture was stirred for 30 minutes while maintaining the temperature. 9.29 g of thiodiglycolic acid was introduced into the reactor while maintaining 0 to 10 °C. After stirring at 0 to 10 °C for three hours, the resulting mixture was filtered to obtain L-erdosteine (yield: 82%).

Although the method for preparing L-erdosteine has been described in detail above, more specific details may be understood with reference to the contents of Korean Registered Patent No. 2583743. Thereby, the entire contents of Korean Registered Patent No. 2583743 are incorporated by reference herein.

### Experimental examples

In the result values of the following experimental examples, L-ERD denotes L-erdosteine, DL-ERD denotes D,L-erdosteine, and D-ERD denotes D-erdosteine.

### Solubility test

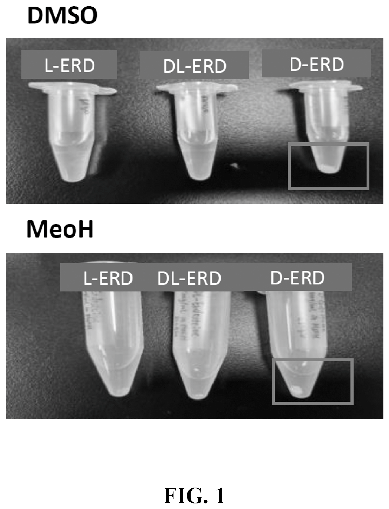

Dimethyl sulfoxide (DMSO) and methanol (MeOH) were used as the solvents, and D-erdosteine exhibited a solubility that was reduced by approximately 90% compared to L,D-erdosteine or L-erdosteine. FIG. 1 is a photograph showing the results of the solubility test.

### Microsomal and S9 stability assay test

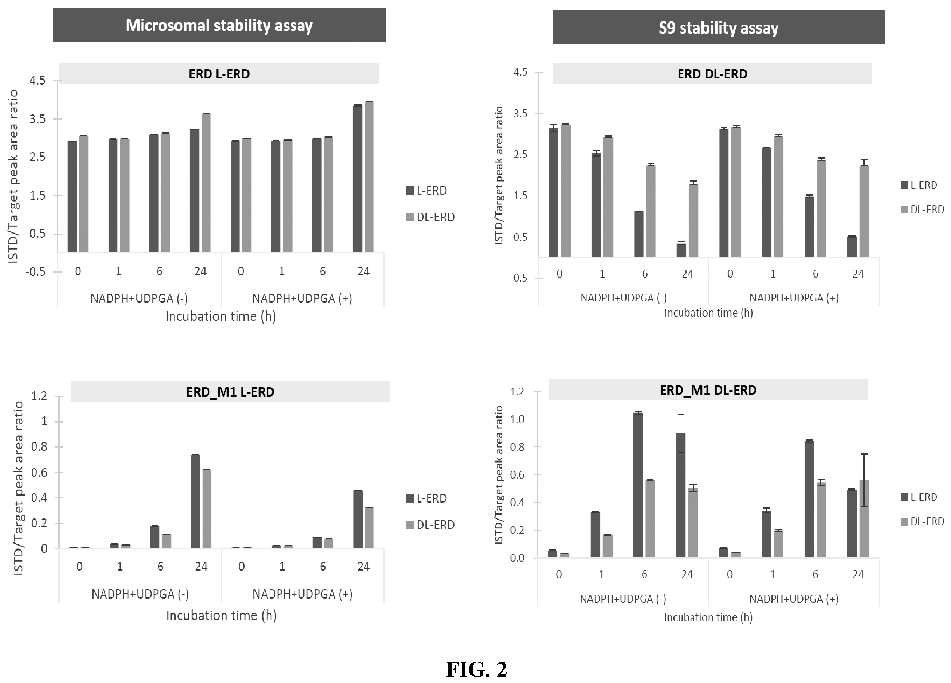

To determine the metabolic stability, microsomal and S9 stability assays were performed. The results are shown in FIG. 2. For reference, the concentration was 100 µM and the reaction temperature was 45 °C, and the reaction temperature did not affect Met-I generation.

When the microsomal stability assay was performed, drug metabolism did not proceed, but upon confirmation of the metabolite, L-erdosteine increased slightly.

In the S9 fraction, in which microsomes and cytosol are mixed, it was confirmed that drug metabolism of erdosteine proceeded, and L-erdosteine was metabolized more rapidly than D,L-erdosteine. The analysis showed that Met-I generated by drug metabolism was also higher for L-erdosteine.

In both *the in vivo* and *in vitro* (S9) assays, it was confirmed that drug metabolism of L-erdosteine proceeds more rapidly than that of D,L-erdosteine, and the generation of Met-I, which has a -SH (sulfhydryl group) released after first-pass metabolism, was also higher for L-erdosteine.

The S9 fraction stability test method mimics the situation occurring in natural liver tissue, and closely reproduces the reactions occurring in actual tissue. In addition, the S9 fraction itself contains various enzymes involved in drug metabolism, and the metabolic process is confirmed through chemical reactions mainly by the cytochrome P450 (CYP) enzymes expressed in hepatocytes. Accordingly, the present inventors carried out a comparative evaluation of the generation of the metabolite M1 of L-erdosteine and racemic erdosteine using rat S9 metabolic stability.

The analytical batch was valid, and as a result of confirming the drug metabolism using the S9 fraction, it was observed that the L-erdosteine-treated group exhibited increased generation of erdosteine metabolite 1 (Met-I) compared to the racemic erdosteine-treated group at the same concentration.

The metabolic stability assay evaluates the metabolic reaction of the target substance, such as the reaction environment composition and reaction promotion, through cofactors (NADPH, UDPGA), but metabolite generation was observed even without cofactor treatment for the test substances L-erdosteine and racemic erdosteine. This appears to be because a chemical reaction due to the metabolic enzymes present in the S9 fraction itself was naturally induced, thereby affecting the drug metabolism, and it was observed that metabolite generation increased with the increase of the test substance concentration and the time from 0 to 6 hours of reaction.

From the above-described results, it was determined that, in the rat S9 fraction stability assay, the test substance L-erdosteine is a compound that increases the formation of the metabolic active substance, erdosteine metabolite 1 (Met-I), compared to racemic erdosteine.

### Pharmacokinetic test I

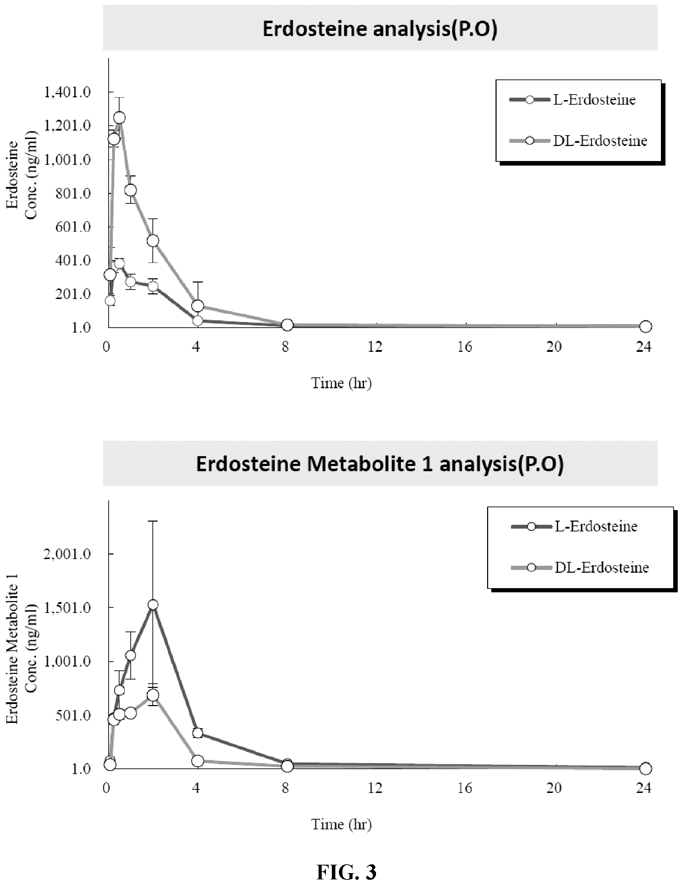

This test was performed to evaluate the pharmacokinetic changes of the active metabolite M1 after single oral administration of L-erdosteine and racemic erdosteine to Sprague-Dawley (SD) rats (test animal: SD rat; test substances: L-erdosteine, D,L-erdosteine; dose (mg/kg/day): 50 mg/kg; excipient: 5% dimethyl sulfoxide (DMSO) + saline; blood-sampling method: jugular venipuncture (heparin)). The results are shown in FIG. 3 and Table 1.

**[Table 1]**

| Parameters | 50 mg/kg | | 50 mg/kg | |
|---|---|---|---|---|
| | L-ERD | DL-ERD | L-ERD-Met-I | DL-ERD-Met-I |
| AUCₗₐₛₜ (ng·hr/ml) | 1092 | 2606 | 4633.4 | 2002.9 |
| AUC_{inf} (ng·hr/ml) | 1173 | 2653.2 | 4739 | 2048.8 |
| Cₘₐₓ | 383.1 | 1251.6 | 1531.3 | 690.3 |
| (ng/ml) | | | | |
| Tₘₐₓ (hr) | 0.5 | 0.5 | 2 | 2 |

As can be seen from the results, L-erdosteine (L-ERD) underwent drug metabolism more rapidly than D,L-erdosteine (DL-ERD), which appears to be a result similar to the above-described *in vitro* assay. In addition, because the metabolism of L-erdosteine proceeded rapidly, the amount of the metabolite after first-pass hepatic metabolism also appears to be higher for L-erdosteine. For reference, when saline at 100 mg/kg was used as an excipient, D,L-erdosteine did not dissolve, so the test was carried out with a combination of 5% DMSO and saline.

As can be confirmed from the *in vitro* and *in vivo* pharmacokinetic test results, the compound of the present invention has a relatively very high area under (blood concentration-time) curve (AUC) of Met-I (Metabolite 1), the erdosteine active metabolite, compared to racemic or D-form erdosteine, and the concentrations of the active metabolite in the blood and tissue can be maintained with only a small amount of intake.

Accordingly, it can be seen that relatively high activity can be exhibited in the target respiratory-organ tissues such as the bronchi and lungs, and furthermore, when the same blood or tissue concentration characteristics are provided, sufficient efficacy can be exhibited with only a small amount of intake, so that a reduction in side effects due to a reduced dose (e.g., gastrointestinal disorders, the most widely known clinical side effect of erdosteine) can be expected.

### Pharmacokinetic test II

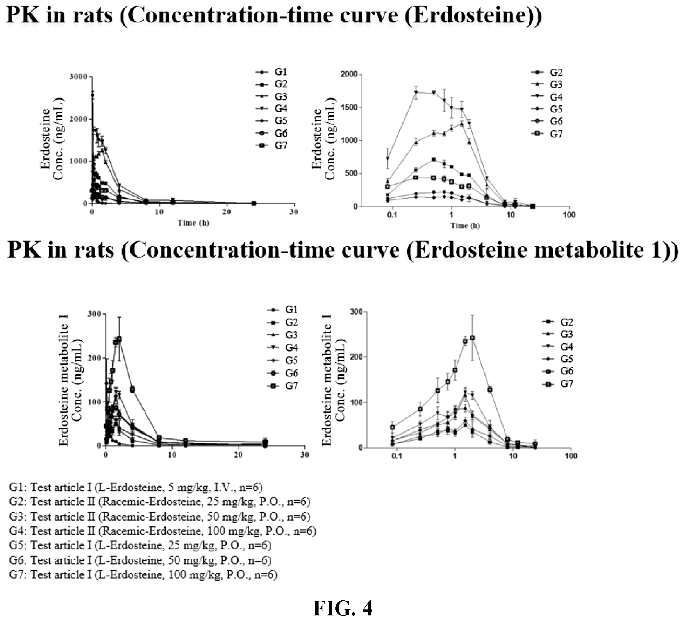

This test was performed to evaluate the pharmacokinetic changes of the active metabolite M1 after single oral administration of L-erdosteine and racemic erdosteine to SD rats (test animal: SD rat; test substances: L-erdosteine, D,L-erdosteine; dose (mg/kg/day): 25 to 100 mg/kg; excipient: 5% DMSO + saline; blood-sampling method: jugular venipuncture (heparin)). The test groups consisted of the L-erdosteine 5 mg/kg I.V. administration group (G1), the racemic erdosteine 25 mg/kg P.O. administration group (G2), the racemic erdosteine 50 mg/kg P.O. administration group (G3), the racemic erdosteine 100 mg/kg P.O. administration group (G4), the L-erdosteine 25 mg/kg P.O. administration group (G5), the L-erdosteine 50 mg/kg P.O. administration group (G6), and the L-erdosteine 100 mg/kg P.O. administration group (G7). Six animals were used per group. The test items included dead animals, general symptom observation, body weight measurement, and PK parameter analysis, and the test results are shown in FIG. 4 and Tables 2 to 5.

**[Table 2]**

| Pharmacokinetic parameters (Erdosteine) | | | | |
|---|---|---|---|---|
| GROUP | AUCₗₐₛₜ (ng*hr/mL) | t_{1/2} (hr) | Cₘₐₓ (ng/mL) | Tₘₐₓ (hr) |
| G1 | 587.80 | 2.10 | 2564.30 | 0.083 |
| G2 | 2318.90 | 3.64 | 715.55 | 0.5 |
| G3 | 4561.99 | 6.01 | 1257.00 | 1.5 |
| G4 | 6335.63 | 2.78 | 1724.70 | 0.25 |
| G5 | 573.78 | 6.22 | 148.65 | 0.75 |
| G6 | 846.43 | 2.96 | 221.94 | 0.75 |
| G7 | 1812.71 | 4.87 | 438.73 | 0.25 |

**[Table 3]**

| Pharmacokinetic parameters (Erdosteine metabolite 1) | | | | |
|---|---|---|---|---|
| GROUP | AUCₗₐₛₜ (ng*hr/mL) | t_{1/2} (hr) | Cₘₐₓ (ng/mL) | Tₘₐₓ (hr) |
| G1 | 63.98 | 0.68 | 142.53 | 0.083 |
| G2 | 169.25 | 6.34 | 49.78 | 1.5 |
| G3 | 409.03 | 5.71 | 116.73 | 1.5 |
| G4 | 506.79 | 6.51 | 122.53 | 1.5 |
| G5 | 233.79 | 4.45 | 59.82 | 1.5 |
| G6 | 426.11 | 4.72 | 86.92 | 1.5 |
| G7 | 1181.10 | 16.75 | 242.81 | 2 |

**[Table 4]**

| Bioavailability of Erdosteine to rat | | | | | |
|---|---|---|---|---|---|
| GROUP | DOSING ROUTE | DOSE (mg/kg) | AUCₗₐₛₜ (ng*hr/mL) | AUCₗₐₛₜ/dose | BIOAVAILABILITY (%) |
| G1 | I.V. | 5 | 587.80 | 117.56 | - |
| G2 | P.O. | 25 | 2318.90 | 92.76 | 78.90 |
| G3 | P.O. | 50 | 4561.99 | 91.24 | 77.61 |
| G4 | P.O. | 100 | 6335.63 | 63.36 | 53.89 |
| G5 | P.O. | 25 | 573.78 | 22.95 | 19.52 |
| G6 | P.O. | 50 | 846.43 | 16.93 | 14.40 |
| G7 | P.O. | 100 | 1812.71 | 18.13 | 15.42 |

| | | | | | |
|---|---|---|---|---|---|
| $Bioavailability \left(F %\right) = \frac{{AUC}_{last} / dose , P . O .}{{AUC}_{last} / dose , I . V .} \times 100$ | | | | | |

**[Table 5]**

| Bioavailability of Erdosteine metabolite 1 to rat | | | | | |
|---|---|---|---|---|---|
| GROUP | DOSING ROUTE | DOSE (mg/kg) | AUCₗₐₛₜ (ng*hr/mL) | AUCₗₐₛₜ/dose | BIOAVAILABILITY (%) |
| G1 | I.V | 5 | 63.98 | 12.80 | - |
| G2 | P.O. | 25 | 169.25 | 6.77 | 52.90 |
| G3 | P.O. | 50 | 409.03 | 8.18 | 63.93 |
| G4 | P.O. | 100 | 506.79 | 5.07 | 39.60 |
| G5 | P.O. | 25 | 233.79 | 9.35 | 73.08 |
| G6 | P.O. | 50 | 426.11 | 8.52 | 66.60 |
| G7 | P.O. | 100 | 1181.10 | 11.81 | 92.30 |

| | | | | | |
|---|---|---|---|---|---|
| $Bioavailability \left(F %\right) = \frac{{AUC}_{last} / dose , P . O .}{{AUC}_{last} / dose , I . V .} \times 100$ | | | | | |

During the observation period, no dead animals were observed, and no notable body weight changes were observed in any of the groups (G1 to G7). As a result of the bioavailability (BA) analysis of erdosteine, the BA of the racemic erdosteine 25 mg/kg P.O., 50 mg/kg P.O., and 100 mg/kg P.O. administration groups was calculated to be 78.90%, 77.61%, and 53.89%, respectively, and the BA of the L-erdosteine 25 mg/kg P.O., 50 mg/kg P.O., and 100 mg/kg P.O. administration groups were calculated to be 19.52%, 14.40%, and 15.42%, respectively. As a result of the BA analysis of erdosteine Met-I, the BA of the racemic erdosteine 25 mg/kg P.O., 50 mg/kg P.O., and 100 mg/kg P.O. administration groups was calculated to be 52.90%, 63.93%, and 39.60%, respectively, and the BA of the L-erdosteine 25 mg/kg P.O., 50 mg/kg P.O., and 100 mg/kg P.O. administration groups was calculated to be 73.08%, 66.60%, and 92.30%, respectively.

In summary, although the BA of L-erdosteine with respect to the parent compound erdosteine was observed to be about 1/4 lower than that of racemic erdosteine, the bioavailability of the active metabolite, Met-I, was observed to be higher for L-erdosteine than for racemic erdosteine, by up to 52.70 percentage points. Therefore, it is determined that L-erdosteine would be more effective than the same dose of racemic erdosteine in the treatment or prevention of various diseases such as acute and chronic respiratory diseases.

### Pharmacokinetic test III

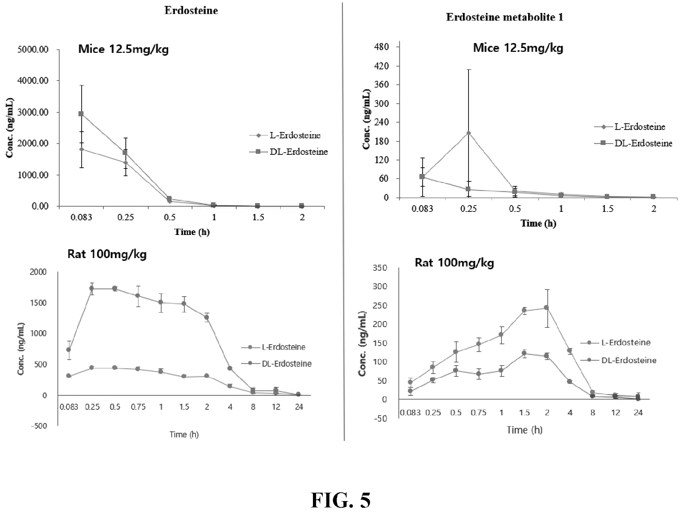

To examine the difference in pharmacokinetic responses between species, a separate pharmacokinetic test was performed (test animals: BALB/c mouse, SD rat; test substances: L-erdosteine, DL-erdosteine; dose (mg/kg/day): BALB/c mouse 12.5 mg/kg, SD rat 100 mg/kg; excipient: 5% DMSO + saline; blood-sampling method: jugular venipuncture (heparin)). The results are shown in FIG. 5.

Although there were differences in the specific shape of the curve, the half-life, and the like between the BALB/c mouse and the SD (Sprague-Dawley) rat species, there was no difference in the tendency for L-erdosteine to raise the concentration of the active metabolite Met-I more rapidly and to a higher level compared to the racemate.

### Evaluation of bronchial relaxation effect of racemic erdosteine and L-erdosteine

This test was performed to evaluate the bronchial relaxation effect when the test substances racemic erdosteine and L-erdosteine were applied to bronchi excised from guinea pigs.

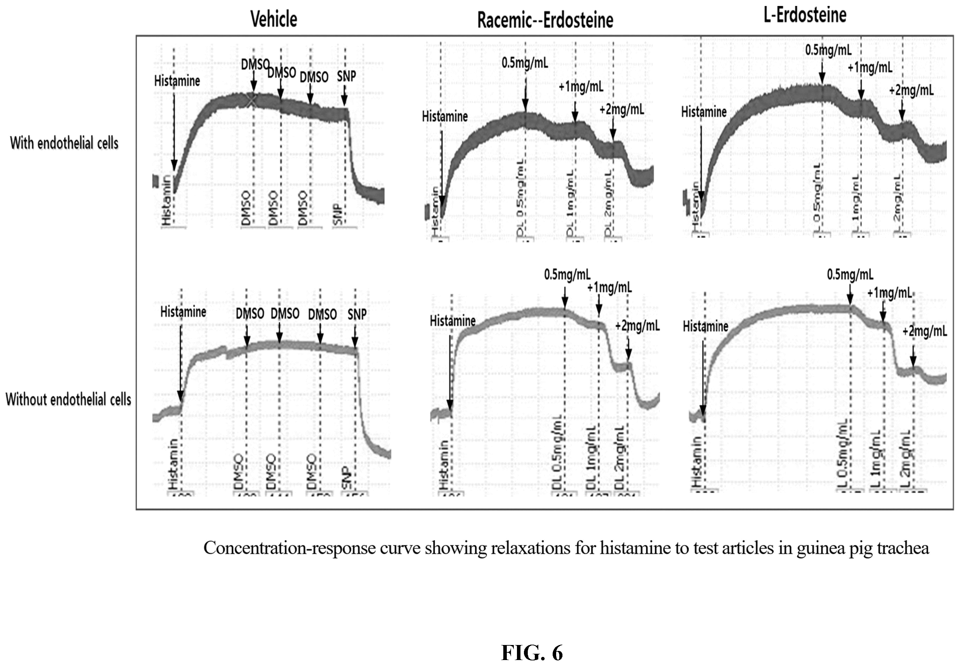

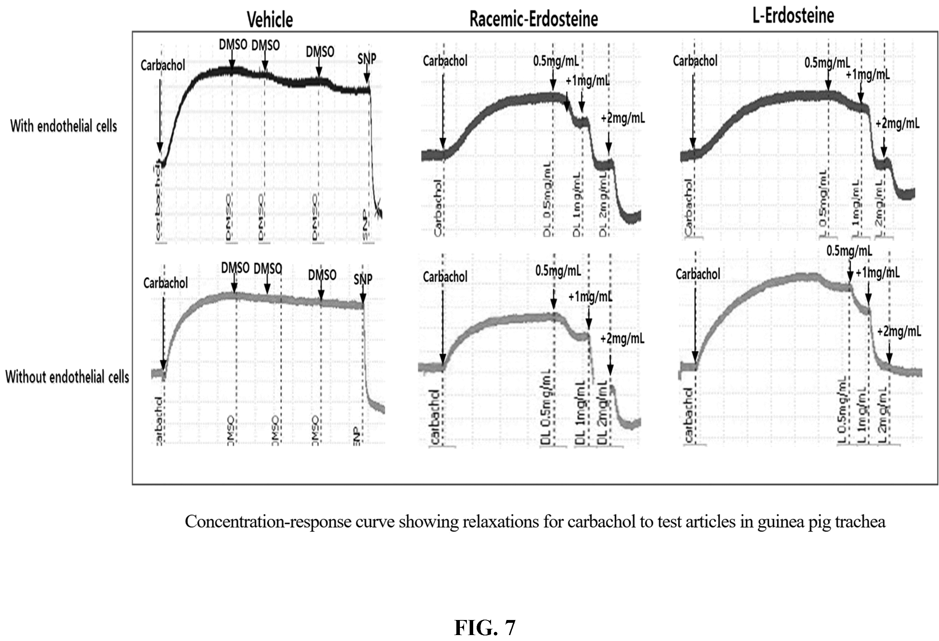

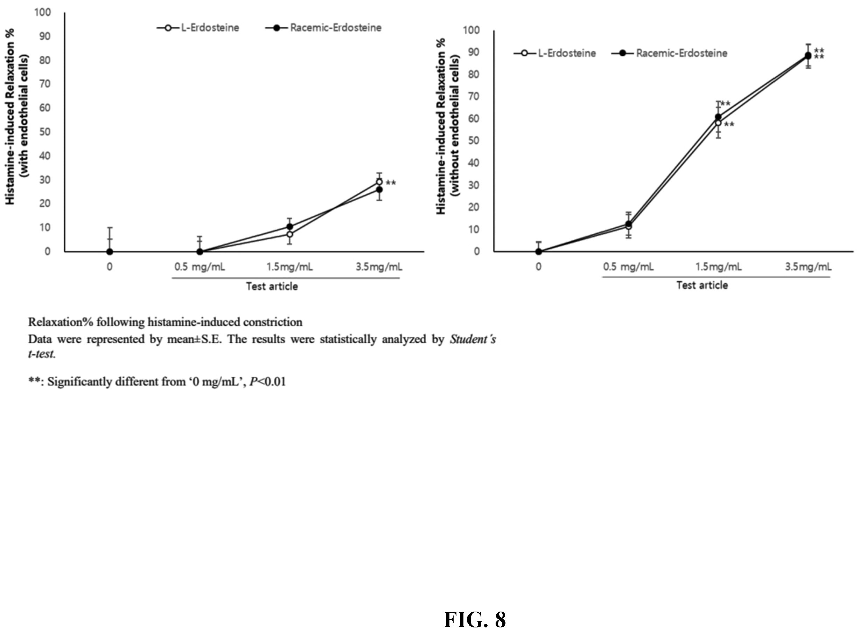

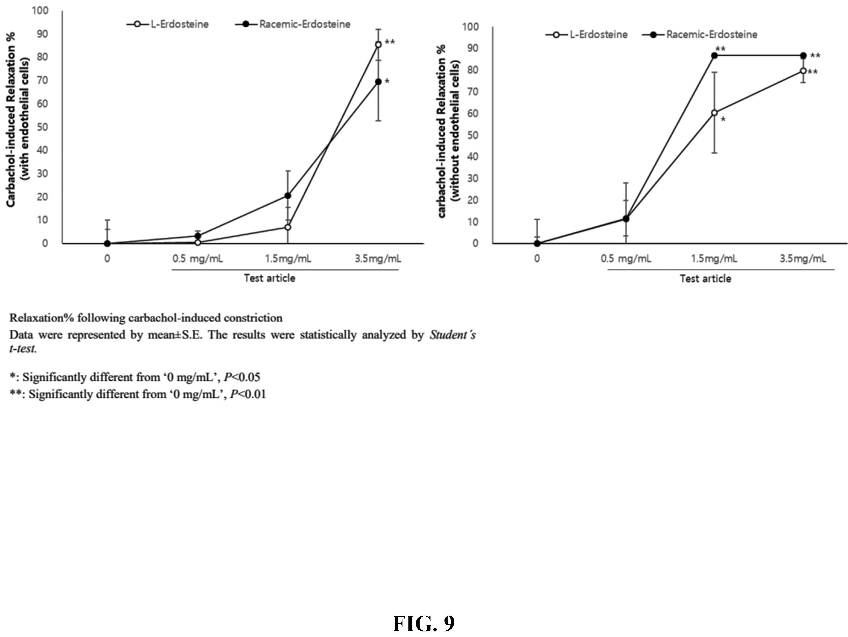

Histamine and carbachol were used as the constrictors for measuring the bronchial relaxation effect, and the treatment concentrations of racemic erdosteine and L-erdosteine were set to final concentrations of 0, 0.5, 1.5, and 3.5 mg/mL for the relaxation effect. The treatment concentration of sodium nitroprusside (SNP), the reference substance for the relaxation effect, was 100 µM. As the test animals, specific pathogen-free (SPF) Hartley guinea pigs (sex: male; age at receipt: 5 weeks; number of animals at receipt: 15; number of animals at organ excision: 7) were used, and the test substance was applied directly to the organ bath containing the excised bronchi at the highest concentration that allowed easy preparation. Histamine (3.2× 10⁻⁵ M) and carbachol (2×10⁻⁷ M) were applied as the constrictors, and when the contraction height reached a plateau, the test substances and the reference substance were applied to observe the relaxation response. The relaxation rate by the test substance was calculated based on the tension when only the baseline tension and the constrictor were applied. For the measurement results, an inter-group comparison was performed using SPSS Statistics 12.0K for medical science, through a Student's t-test, which is a parametric multiple comparison procedure. A comparison between the excipient control group and the test substance-administration group, and a comparison between the test substances racemic erdosteine and L-erdosteine, were performed, and a P-value of less than 0.05 was determined to be significant. To confirm the half-maximal effective concentration (EC₅₀), the Probit method was performed. The test results are shown in FIGS. 6 to 9.

In histamine-induced airway contraction, a significant increase in the relaxation rate was observed upon treatment with L-erdosteine at 3.5 mg/mL, and an increasing tendency was observed upon treatment with racemic erdosteine at 3.5 mg/mL. In carbachol-induced airway contraction, a significant increase in the airway relaxation rate was observed upon treatment with racemic erdosteine and L-erdosteine at 3.5 mg/mL.

In the relaxation rate measurement after the removal of endothelial cells, performed to confirm the effect on the airway endothelial cells, a significant increase in the airway relaxation rate by the constrictors histamine and carbachol was observed in the treatments with racemic erdosteine and L-erdosteine at the 1.5 mg/mL and 3.5 mg/mL.

The EC₅₀ measured for airway relaxation against histamine-induced contraction was 4.61 mg/mL for racemic erdosteine and 4.34 mg/mL for L-erdosteine, and the EC₅₀ measured for airway relaxation against carbachol-induced contraction was 2.77 mg/mL for racemic erdosteine and 2.66 mg/mL for L-erdosteine. After the removal of the airway endothelial cells, the EC₅₀ measured for airway relaxation against histamine-induced contraction was 1.76 mg/mL for racemic erdosteine and 1.82 mg/mL for L-erdosteine, and the EC₅₀ measured for airway relaxation against carbachol-induced contraction was 1.45 mg/mL for racemic erdosteine and 1.93 mg/mL for L-erdosteine.

From the above-described results, it is determined that the airway relaxation rate effect against histamine and carbachol is equivalent for racemic erdosteine and L-erdosteine, regardless of the presence or absence of the airway endothelial cells.

### Expectorant effect test of L-erdosteine and racemic erdosteine

Using NCI-H292 cells (tissue: lung; medium: RPMI1640; supplier: Korean Cell Line Bank), the MUC5AC-production effect of the test substances L-erdosteine and racemic erdosteine by concentration was evaluated.

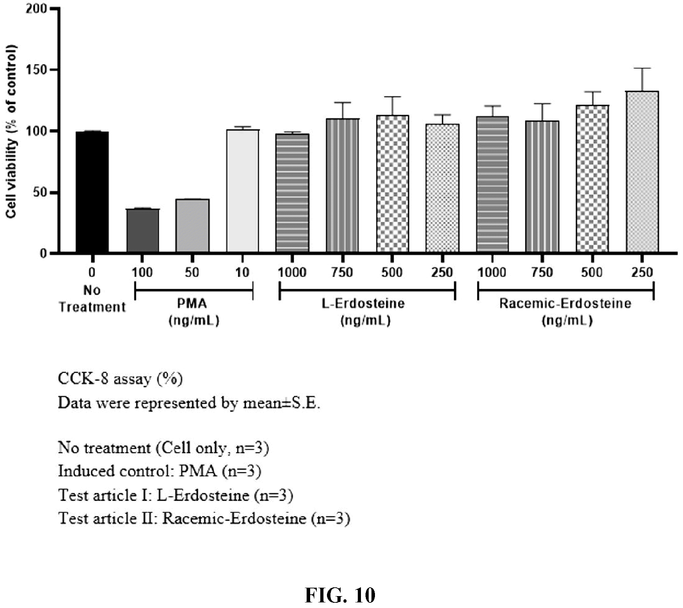

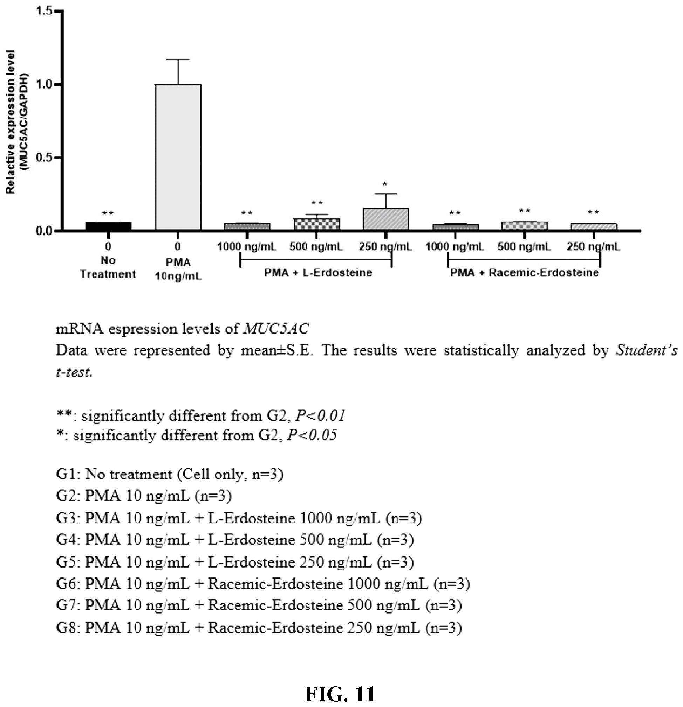

The test substances L-erdosteine and racemic erdosteine were each prepared by dissolving in methanol at a concentration of 10.6 mg/mL, and the inducer was prepared by dissolving phorbol-12-myristate-13-acetate (PMA) in DMSO at a concentration of 1 mg/mL. SPSS Statistics 12.0K for medical science was used for the analysis of the measurement results. A Kolmogorov-Smirnov test confirmed that all data were normally distributed, and the induced control group (G2) was compared with the untreated group (G1) and the test substance-treated groups (G3 to G8) through a parametric multiple comparison procedure. A Student's t-test was performed as the analysis method, and a Welch's t-test was performed in the case of unequal variance. All statistical analyses were determined to be statistically significant when P<0.05. The MUC5AC expression level was evaluated using quantitative PCR (qPCR). In addition, cytotoxicity by concentration (CCK-8 assay) was also measured, and the concentration at which the cell survival rate in the NCI-H292 cells was 80% or more was set as the concentration without cytotoxicity. The results are shown in FIG. 10 (cytotoxicity) and FIG. 11 (mRNA expression level of MUC5AC).

No cytotoxicity was observed at the concentration of PMA 10 ng/mL, and L-erdosteine and racemic erdosteine were observed to be without cytotoxicity at all concentrations.

Meanwhile, the inducer PMA 10 ng/mL-treated group (G2) showed a significant increase in MUC5AC mRNA expression compared to the untreated group (G1); the L-erdosteine 1000 ng/mL, 500 ng/mL, and 250 ng/mL-treated groups (G3 to G5) showed a statistically significant decrease in MUC5AC mRNA expression compared to the inducer PMA 10 ng/mL-treated group (G2); and the racemic erdosteine 1000 ng/mL, 500 ng/mL, and 250 ng/mL-treated groups (G6 to G8) showed a statistically significant decrease in MUC5AC mRNA expression compared to the inducer PMA 10 ng/mL-treated group (G2).

From these results, it was confirmed that, in the NCI-H292 cells, MUC5AC production increased due to PMA, and the expression level of MUC5AC decreased when treated with L-erdosteine and racemic erdosteine at concentrations of 1000 ng/mL, 500 ng/mL, and 250 ng/mL, and thus L-erdosteine and racemic erdosteine were determined to be effective for expectoration.

### Pulmonary inflammation-inhibitory effect test of L-erdosteine and racemic erdosteine

An evaluation of the pulmonary inflammation-inhibitory effect was performed when L-erdosteine and racemic erdosteine were repeatedly orally administered for 21 days to a bleomycin-induced lung injury mouse model.

Each test substance of L-erdosteine and racemic erdosteine was weighed by dose, sequentially added at an excipient ratio of DMSO:saline=5.0:95.0 (v/v), and vortexed to prepare, and the resulting preparation was administered. The test animals used were a mouse model (SPF C57BL/6N mice, C57BL/6NCrljOri; sex: male; age: 7 weeks; body weight range: 19.41 to 22.79 g). The administration method was oral administration (number of administrations: once/day for 21 days, 21 administrations in total; administration method: the dorsal cervical region of the animal was fixed by hand, and the substance was administered directly into the stomach using a 1 mL syringe equipped with a zonde; administration volume: 10 ml/kg/day). Lung injury was induced by incising the skin along the center of the neck to expose the upper airway, and injecting bleomycin dissolved in physiological saline injection solution into the lung in a spray form through the upper airway at a dose of 1.25 mg/kg (50 µL) using a specially manufactured syringe, followed by suturing. Body weight was measured at the start of administration, and thereafter twice/day and on the day of necropsy.

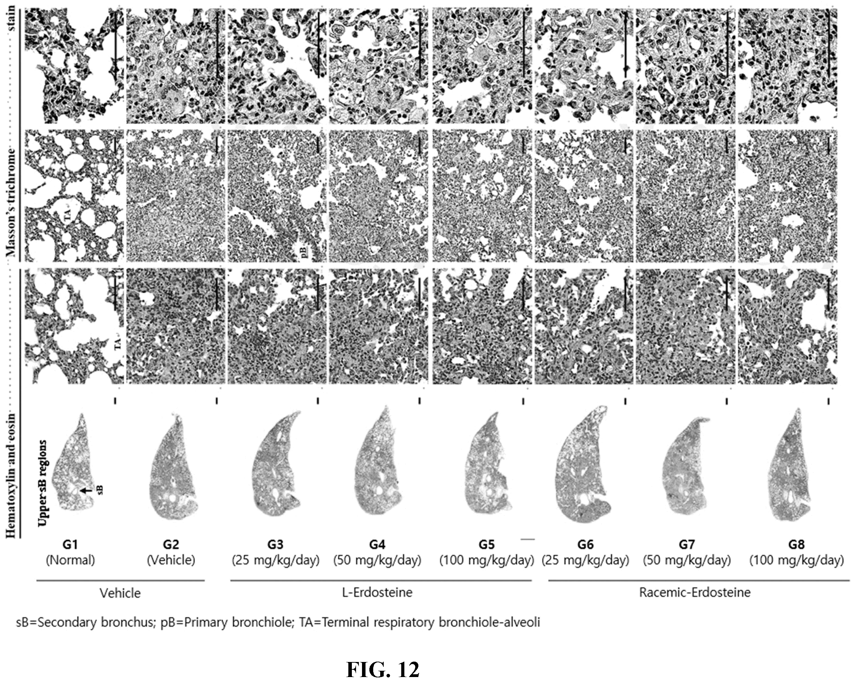

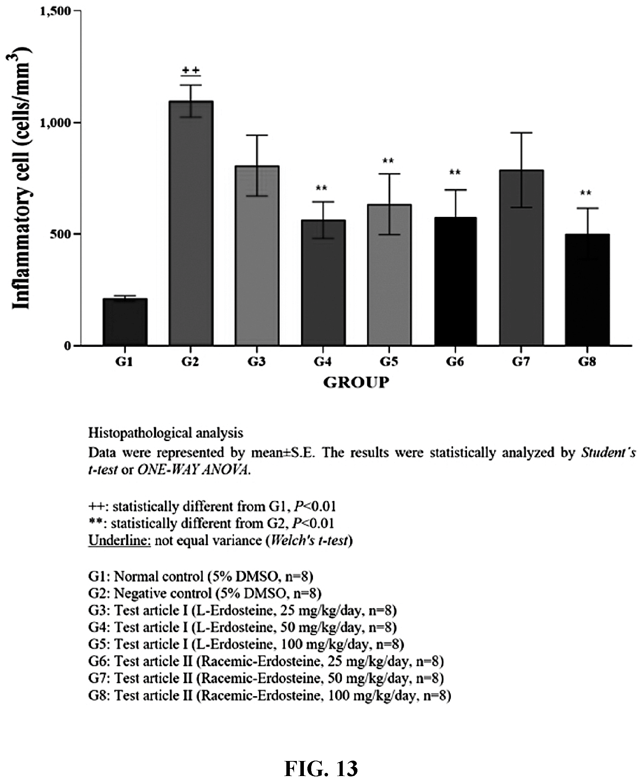

The test groups consisted of a normal group (G1), a negative control group (G2), a test substance L-erdosteine 25 mg/kg/day administration group (G3), an L-erdosteine 50 mg/kg/day administration group (G4), an L-erdosteine 100 mg/kg/day administration group (G5), a test substance racemic erdosteine 25 mg/kg/day administration group (G6), a racemic erdosteine 50 mg/kg/day administration group (G7), and a racemic erdosteine 100 mg/kg/day administration group (G8). Eight animals were used per group. The test items included dead animals, general symptoms, and observation of body weight, and histopathological examination (the left lobe of the animal was excised on the day of necropsy, observed and photographed using an optical microscope (Nikon, Japan) and a camera (Jena, Germany), and the inflammatory cell numbers were analyzed). The statistical analysis used SPSS Statistics 12.0K for medical science. The comparison of the normal group (G1) and the negative control group (G2) in the parametric comparison procedure was an inter-group comparison to determine the presence or absence of disease induction. A Student's t-test was performed as the analysis method, and a Welch's t-test was performed in the case of unequal variance. The comparison between the negative control group (G2) and the test substance-administered groups (G3 to G8) was performed by ONE-WAY ANOVA, and as post-hoc testing, a Duncan's test was performed in the case of equal variance and a Dunnett's test was performed in the case of unequal variance. All statistical analyses were determined to be statistically significant when P<0.05. The test results are shown in FIG. 12, FIG. 13, and Table 6.

**[Table 6]**

| | HISTOPATHOLOGICAL ANALYSIS | MALE |
|---|---|---|
| GROUP | INFLAMMATORY CELL (cells/mm²) | |
| G1 | 214.00±12.07 | |
| G2 | 1096.25±70.91= | |
| G3 | 806.75±136.50 | |
| G4 | 562.50±81.79** | |
| G5 | 634.25±136.63** | |
| G6 | 579.50±118-68** | |
| G7 | 787.00±167.70 | |
| G8 | 501.00±114.67** | |

| | | |
|---|---|---|
| Data were represented by mean±S.E. The results were statistically analyzed by *Student's t-test* on *ONE-WAY ANOVA.* ++: statistically different from G1, *P*<0.01 **: statistically different from G2, *P<*0.01 Underline: not equal variance *(Welch's t-test* or *Dunnett post-hoc test)* G1: Normal control (5% DMSO, n=8) G2: Negative control (5% DMSO, n=8) G3: Test article I (L-Erdosteine, 25 mg/kg/day, n=8) G4: Test article I (L-Erdosteine, 50 mg/kg/day, n=8) G5: Test article I (L-Erdosteine, 100 mg/kg/day, n=8) G6: Test article II (Racemic-Erdosteine, 25 mg/kg/day, n=8) G7: Test article II (Racemic-Erdosteine, 50 mg/kg/day, n=8) G8: Test article II (Racemic-Erdosteine, 100 mg/kg/day, n=8) | | |

During the test period, no abnormal symptoms or dead animals were observed, and no inter-group difference in body weight was observed for the test substance-administered groups (G3 to G8) compared to the negative control group (G2).

In the number of inflammatory cells, a statistically significant decrease was observed for the L-erdosteine 50 and 100 mg/kg/day administration groups (G4 and G5) and the racemic erdosteine 25 and 100 mg/kg/day administration groups (G6 and G8) compared to the negative control group (G2).

From the above-described results, it was confirmed that the 21-day repeated oral administration of L-erdosteine and racemic erdosteine in the bleomycin-induced lung injury mouse model has an effect of inhibiting the pulmonary inflammation response, and furthermore, since pulmonary fibrosis occurs as an inflammatory response such as pneumonia progresses over a long period, a long-term pulmonary fibrosis-preventive effect can also be expected.

### Respiratory inflammation-alleviating effect test of L-erdosteine and racemic erdosteine

A comparative evaluation of the respiratory inflammation-alleviating effect by three repeated oral administrations of L-erdosteine and one other substance to ovalbumin (OVA; albumin from chicken egg white)-induced asthma BALB/c mouse model was performed.

L-erdosteine and racemic erdosteine were used as the test substances; OVA and alum (aluminum hydroxide) were used as the inducers; and dexamethasone was used as the positive control substance. Each test substance and the positive control substance were weighed by dose, and the test substances were sequentially added at an excipient ratio of DMSO:saline=5.0:95.0 (v/v), the positive control substance was added with water for injection, and they were vortexed to prepare. The test animals used were a mouse model (SPF C57BL/6N mice, C57BL/6NCrljOri; sex: male; age: 9 weeks; body weight range: 21.38 to 26.18 g), and the administration method was oral administration (number of administrations: once/day for 3 days, 3 administrations in total; administration method: the dorsal cervical region of the animal was fixed by hand, and the substance was administered directly into the stomach using a 1 mL syringe equipped with a zonde; administration volume: 10 ml/kg/day). Body weight was measured at the start of administration, and thereafter twice/week and on the day of necropsy.

Asthma induction was performed according to the following procedure.

### 1. Sensitization

**1.1.** After acclimatization of the Cohort 1 animals for 4 days and the Cohort 2 animals for 11 days, sensitization was performed by intraperitoneally administering a total of two doses of the sensitization solution OVA 50 µg/0.1 mL/head on day -20 and day -6. The normal group was administered the excipient in the same manner.

### 1.2. Preparation of sensitization solution

1.2.1. OVA (grade V chicken egg OVA (Sigma-Aldrich, St. Louis, MO, USA)) was taken out of the refrigerator and allowed to stand at room temperature for at least 30 minutes, then dissolved by adding phosphate-buffered saline (PBS) to a concentration of 10 mg/mL, and 1 mL of this solution and 9 mL of PBS were mixed to prepare a diluted OVA stock solution (1 mg/mL).

1.2.2. 7.5 mL of the OVA stock solution and 7.5 mL of alum were placed in a 50 mL conical tube (0.5 mg/mL), mixed, and mixed well for 30 minutes using a shaker to complete the sensitization solution.

### 2. Challenge

2.1. The challenge was performed on day 1, 2, and 3 (3 times in total).

2.2. One hour after test substance administration, 1% OVA 10 mL (10 mg/mL) was sprayed into a chamber to perform the challenge.

2.3. The challenge was performed for 60 minutes over a total of 3 days on day 1, 2, and 3, and test substance administration and challenge were performed simultaneously for three days.

2.4. At this time, one or two animals of each test group were placed in the same chamber, the lid was covered, and a silicone hose connected to a nebulizer was inserted into a hole in the cage to spray.

2.5. The normal group was sprayed with the excipient in the same manner.

### 2.6. Preparation of challenge solution

**2.6.1.** OVA (grade III OVA (Sigma-Aldrich)) was taken out of the refrigerator and allowed to stand at room temperature for at least 30 minutes, then 600-800 mg was weighed into a 50 mL conical tube.

**2.6.2.** OVA was dissolved by adding PBS to a concentration of 10 mg/mL, then placed on ice to maintain a refrigerated state.

The test groups consisted of a normal group (G1), an excipient control group (G2), an L-erdosteine 12.5 mg/kg/day administration group (G3), a 25 mg/kg/day administration group (G4), a 50 mg/kg/day administration group (G5), a racemic erdosteine 12.5 mg/kg/day administration group (G6), a 25 mg/kg/day administration group (G7), a 50 mg/kg/day administration group (G8), and a dexamethasone 3 mg/kg/day administration group (G9, hereinafter the positive control group). Eight animals were used per group. The statistical analysis was performed using SPSS Statistics 12.0K for medical science. The comparison of the normal group (G1) and the excipient control group (G2) in the parametric comparison procedure was an inter-group comparison to determine the presence or absence of asthma induction. The comparison of the excipient control group (G2) and the test substance-administered groups (G3 to G8) was performed by ONE-WAY ANOVA, and as post-hoc testing, a Duncan's test was performed in the case of equal variance and a Dunnett's test was performed in the case of unequal variance. The comparisons between the normal group (G1) and the positive control group (G9), and between the excipient control group (G2) and the L-erdosteine-administered groups (G3 to G5) and the racemic erdosteine-administered groups (G6 to G8), were performed by a Student's t-test, and by a Welch's t-test in the case of unequal variance. In addition, statistical exclusion of the western blot measurement results was performed using a box-plot. All statistical analyses were determined to be statistically significant when P<0.05.

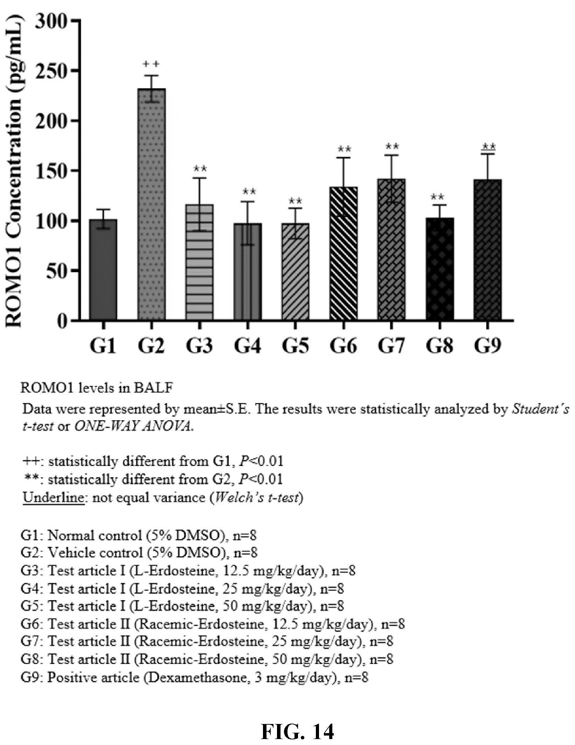

As the test items, dead animals and general symptoms were observed (death during the administration and observation period, the day of symptom onset, and the degree thereof were observed once a day and recorded for each individual, with the administration start day taken as day 1); body weight was measured (at receipt and at group separation, and thereafter twice/week and on the day of necropsy); and reactive oxygen species modulator 1 (ROMO1) was analyzed for the bronchoalveolar lavage fluid (BALF) using an ELISA Kit (LS Bio, Cat. LS-F35358). The test results are shown in FIG. 14.

During the test period, no abnormal symptoms or dead animals were observed. No inter-group difference in body weight was observed in any of the administration groups (G1 to G9). A decreasing tendency of ROMO1 in the BALF was observed for the L-erdosteine 12.5 mg/kg/day and 25 mg/kg/day administration groups (G3 and G4) compared to the racemic erdosteine 12.5 mg/kg/day and 25 mg/kg/day administration groups (G6 and G7).

From the above-described results, in the OVA-induced asthma mouse model, a decrease in ROMO1 by the three oral administrations of L-erdosteine and racemic erdosteine was observed, and thus it is determined that L-erdosteine is more effective than the same dose of racemic erdosteine in regulating and improving respiratory-related chronic inflammatory diseases such as chronic upper respiratory tract inflammation, asthma, and chronic obstructive pulmonary disease (COPD). For reference, it is reported that the serum ROMO1 concentration in COPD patients is significantly higher than the serum ROMO1 of a healthy control group (Liang et al., "Increased Serum Romo1 Was Correlated with Lung Function, Inflammation, and Oxidative Stress in Chronic Obstructive Pulmonary Disease", Inflammation. 2019 Oct;42(5):1555-1560. doi: 10.1007/s10753-019-01017-x.)

## Claims

1. A use of an L-erdosteine compound represented by Chemical Formula 1 below in the manufacture of a medicament for one or more selected from mucolysis and expectoration in acute and chronic respiratory diseases, treatment or amelioration of upper respiratory tract inflammation, treatment or prevention of acute and chronic bronchitis, treatment or prevention of cough, treatment or prevention of asthma, treatment or prevention of pneumonia, prevention of pulmonary fibrosis, prevention of bronchial and alveolar destruction, treatment or prevention of chronic obstructive pulmonary disease, increase of resistance to bacterial infections of upper respiratory tract, and amelioration of side effects caused by smoking:

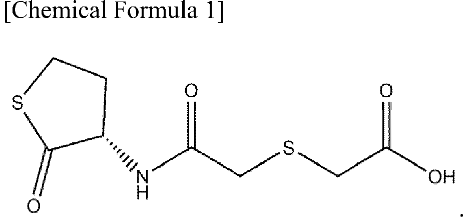

2. A use of an L-erdosteine compound as a prodrug of a compound represented by Chemical Formula 2 below:

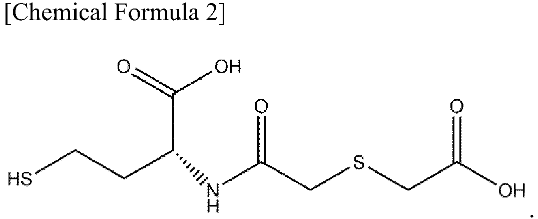

3. A pharmaceutical composition comprising an L-erdosteine compound represented by Chemical Formula 1 below as an active ingredient, for one or more uses selected from mucolysis and expectoration in acute and chronic respiratory diseases, treatment or amelioration of upper respiratory tract inflammation, treatment or prevention of acute and chronic bronchitis, treatment or prevention of cough, treatment or prevention of asthma, treatment or prevention of pneumonia, prevention of pulmonary fibrosis, prevention of bronchial and alveolar destruction, treatment or prevention of chronic obstructive pulmonary disease, increase of resistance to bacterial infections of upper respiratory tract, and amelioration of side effects caused by smoking:

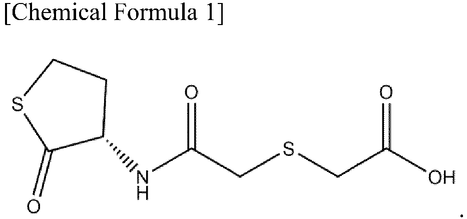

4. The pharmaceutical composition of claim 3, wherein the pharmaceutical composition includes the L-erdosteine compound at a daily dose of 10 to 200 mg/kg.

5. The pharmaceutical composition of claim 3, wherein the pharmaceutical composition includes 50 to 200 mg of the L-erdosteine compound and a pharmaceutically acceptable excipient.
